# EUROPEAN PATENT SPECIFICATION

(11) **EP 1 286 710 B1**
(45) Date of publication and mention of the grant of the patent: **30.03.2005**
(21) Application number: 01936626.9
(22) Date of filing: 31.05.2001
(51) Int. Cl.: A61M 5/142

(54) **SUPER ABSORBENT DRIVEN DISPENSER**
SUPERABSORBER GETRIEBENE AUSGABEVORRICHTUNG
DISTRIBUTEUR ACTIONNE PAR UN MATERIAU SUPERABSORBANT

(30) Priority: 01.06.2000 GB 0013281
(43) Date of publication of application: 05.03.2003
(73) Proprietor: Sonoco Absorbent Technologies LLC., Horndean, Hampshire PO8 9JU (GB)
(72) Inventor: DAVIDSON, Roderick Iain, West Meon, Petersfield, Hampshire GU32 (GB); PRIVETT, Rupert Kenneth, Horndean, Hampshire PO8 0AB (GB); BOYD-MOSS, Graeme Stuart Durban, Andover, Hampshire SP11 8EL (GB)
(74) Representative: Brooks, Nigel Samuel
(86) International application number: PCT/GB2001/002406
(87) International publication number: WO 2001/091832

(56) References cited:
- WO-A-98/37959
- GB-A- 1 385 272

## Description

The present invention relates to super-absorbent materials and their use in providing a motive force on expansion during uptake of liquid.

The use of osmotic pressure has been suggested as a way of dispensing drug in GB-A-1385272. The device comprises a first impermeable, flexible container, and a second water permeable, flexible container, within a non-flexible, permeable housing. As water permeates though the housing, it also permeates though the second container wall, thereby increasing the volume of the second container. As the housing is of a fixed volume, the increase in volume of the second container presses upon the first container, dispensing the contents thereof. However, such a system is very difficult to control and has a narrow range of uses.

Super-absorbent materials are widely used due to their ability to absorb many times their own weight in liquid. In particular they are used in nappies, meat pads, and packaging where liquid spills must be avoided.

As the super-absorbent materials take in liquid, they expand. Such is their affinity with water, that they can exert a substantial force when expanding. We propose to harness this force, which can be used for displace other objects, for example to move a piston, or to displace a volume of liquid. As the expansion is controlled by the volume of water added to the super-absorbent material, the expansion of the super-absorbent material can be used to dose or meter the rise in the piston, or the volume of liquid displaced.

The object of the present invention is to provide an actuator for exerting a motive force using super-absorbent material materials.

According to the invention there is provided a device for displaying material by movement of a piston or diaphragm, the device comprising:
- an enclosure containing unsaturated super-absorbent material and including a piston or diaphragm; and
- a fluid inlet into the enclosure adapted to allow fluid into the enclosure and restrain the super-absorbent material from escaping from the enclosure;
the arrangement being such that as fluid is allowed into the enclosure, it is absorbed by the super-absorbent material which swells against the piston or diaphragm, causing movement and displacement of the material.

In one embodiment the piston or diaphragm is a membrane of elastomeric material diving a first section of the enclosure from a second section, one for holding the unsaturated super-absorbent material, and the other for holding liquid or the like to be displaced. In a further embodiment the diaphragm is a bag forming a second section within the enclosure, sealed at or near an outlet of the enclosure, the bag being fillable with a liquid to be displaced.

Typically the volume of liquid will be controlled to determine the expansion of the super absorbent material and thus the force exerted. Additionally or alternatively the speed of the expansion can be controlled by controlling the purity of the water. The use of pure water will be absorbed more rapidly than water containing contaminants. Thus controlling the quantity of contaminants will control the speed of absorption of the water and hence the force exerted.

The super-absorbent material may be in granular or fibrous form depending upon the application.

The enclosure can be an extension of a cap into a body, the body being adapted to hold water, and the water inlet being between the extension and a base of the body on lifting of the extension.

Alternatively the enclosure can be a section of a container, with the movable wall being a diaphragm dividing the container. In this embodiment the water inlet is a wick connecting the enclosure to a bowl for measuring a predetermined volume of water.

In a further alternative super-absorbent material actuator may be part of a drug delivery system. In this embodiment the diaphragm is an elastic membrane and the material is liquid in the elastic membrane.

To help understanding of the present invention three embodiments thereof will now be described with reference to the accompanying drawings in which:-
Figure 1 is a front view of a pouch having a front section containing liquid and a back section containing super-absorbent material material;
Figure 2 is a cross section of the pouch of Figure 1 according to the present invention;
Figure 3 is a cross section of the pouch of Figure 1 after it has been exposed to water;
Figure 4 is a cross section of an air freshener according to the present invention prior to use;
Figure 5 is a cross section of the air freshener of Figure 4 in use;
Figure 6 is a cross section of a dispenser according to a further aspect of the present invention;
Figure 7 is a cross section of the dispenser of Figure 6 during use;
Figure 8 is a cross section of a disposable drug infusion pump according to a third aspect of the present invention.

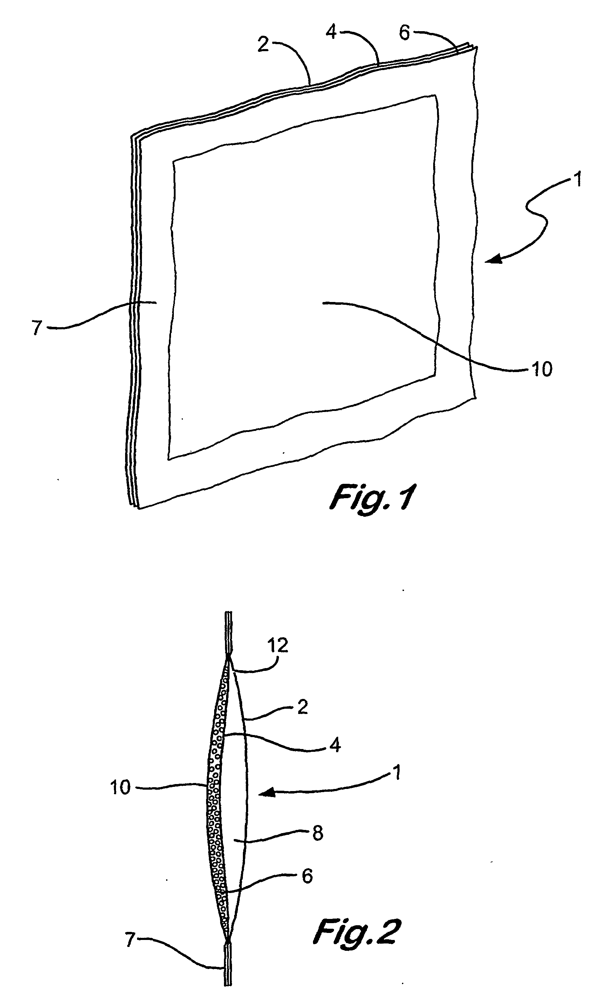

Referring first to Figures 1 and 2, the pouch 1 thereshown is designed to discharge a liquid, when exposed to water. The pouch has three layers, 2, 4, 6 bonded together at edges 7 thereof. The first outer layer 2 is water impervious and the second central layer 4, is also water impervious. Between these two layers, 2, 4, there is captured a quantity of liquid, 8. The third outer layer, 6 is water permeable, and between this third layer 6 and the central layer 4 is captured some super-absorbent material 10. The super-absorbent material may be in any form, for example granules, as shown, or fibrous form. Before use a small hole 12, for example a pin prick, is made in the first outer layer, which does not pierce any of the other layers.

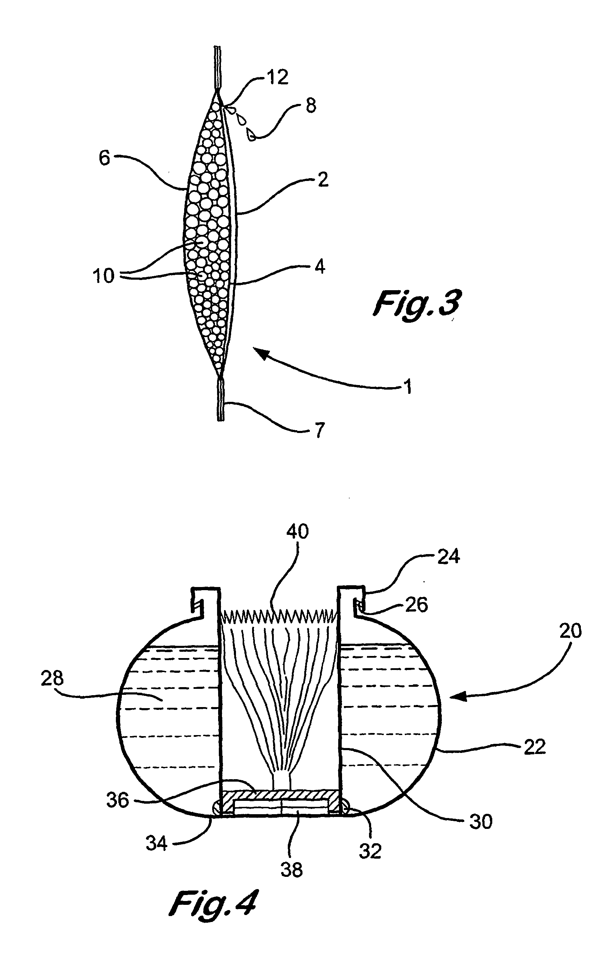

Referring now to Figure 3, when water comes into contact with the third, water permeable layer 6, it passes through this layer 6 and is absorbed by the super-absorbent material captured between the third layer 6 and the central layer 4. As it absorbs the super-absorbent material expands and pushes on the layers of the pouch 1. This results in a reduction in the size of the space between the first and second layers, 2, 4, and thus forces the liquid 8, out of the pouch 1 through the hole 12. Thus the force generated by the expansion of the super-absorbent material has displaced the liquid from the pouch.

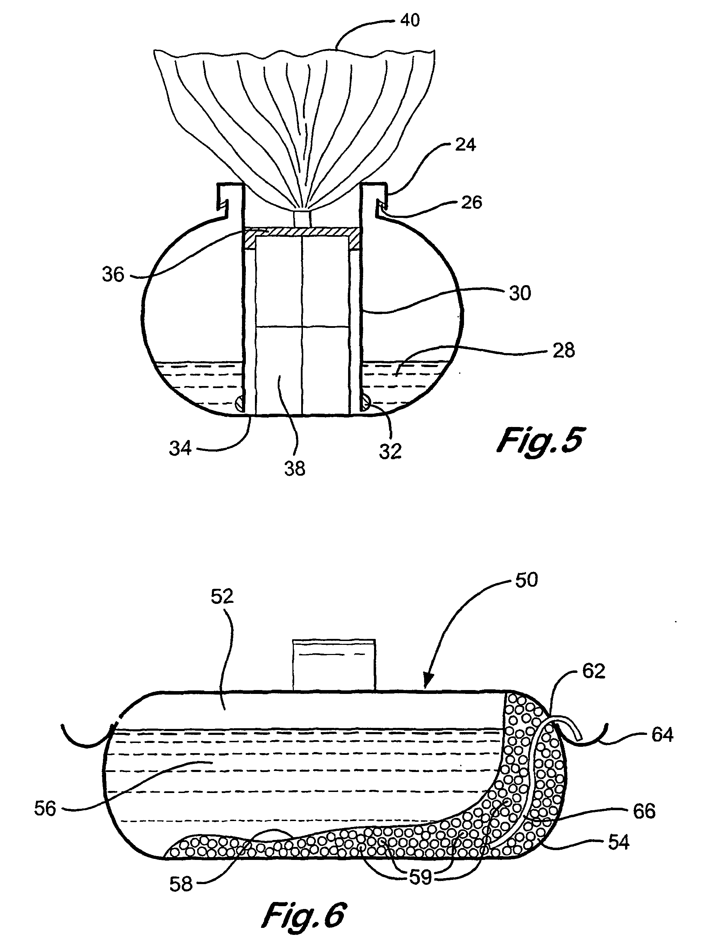

Referring now to Figures 4 and 5, the device 20 thereshown is an air freshener, which produces flower by means of the actuator of the invention. The device 20 comprises a body 22, which holds a quantity of the water 28 including perfume or deodorising chemicals. The device also includes a cap 24, which is attached to the body 22 and movable between an upper position and a lower position by means of rotation on a rising cam surface 26. The cap includes an extension, which extends from the cap 24 into the body 22 and is sized such that a seal 32 on the distal end of the extension, seals onto a base 34 of the body 22 when the cap 24 is in the lower position.

Inside the extension is a piston 36 having a perforated surface, arranged such that it can slide up and down the length of the extension 30. Underneath the piston 36 is provided some super-absorbent material 38, usually in the form of one or more pads. On top of the piston 36 is mounted an artificial flower 40, although any from of display item could be used, for example a Christmas tree or Santa. Prior to use, the piston 36 and flower 40 are pushed as far as possible into the extension 30.

In use, the cap 24 is twisted, raising the cap to its upper position. This raises the seal 32 from firm contact with the base 34 of the body allowing the water 28 into the extension 30. As the water seeps into the extension 30, it comes into contact with the super-absorbent material 38 and is absorbed thereby. This results in expansion of the super-absorbent material 38, which is underneath the piston 36, and thus causes the piston to rise in the extension. More water then seeps into the extension 30 and is absorbed by the super-absorbent material 38, causing the piston 36 to rise further. This continues until the super-absorbent material has absorbed the maximum volume of water possible, i.e. it is saturated, or until all the water has been used. Due to the perforations in the piston, there will be evaporation of the water and chemicals contained therein to the atmosphere of the room.

As the piston 36 rises through the extension 38, it raises the flower 40, which when unrestricted by the confines of the extension, opens. The rate of rise of the piston 36, and thus growth of the flower 40, is determined by the expansion of the super-absorbent material 38, which in term is governed by the rate at which water can enter the extension and the purity of the water. The purer the water, the faster its uptake by the super-absorbent material and this the faster the rate of expansion of the super-absorbent material. However, as the device is designed to emit a perfume into the atmosphere the water will contain perfumes and deodorisers.

To increase the rate of evaporation of the water, a wick may be provided, not shown, to wick the water from the super-absorbent material onto the petals of the flower. In addition a colorant may be added which colours the petals of the flowers while there is remaining water. As the air freshener becomes useless, the colour will fade, indicating that the air freshener should be replaced.

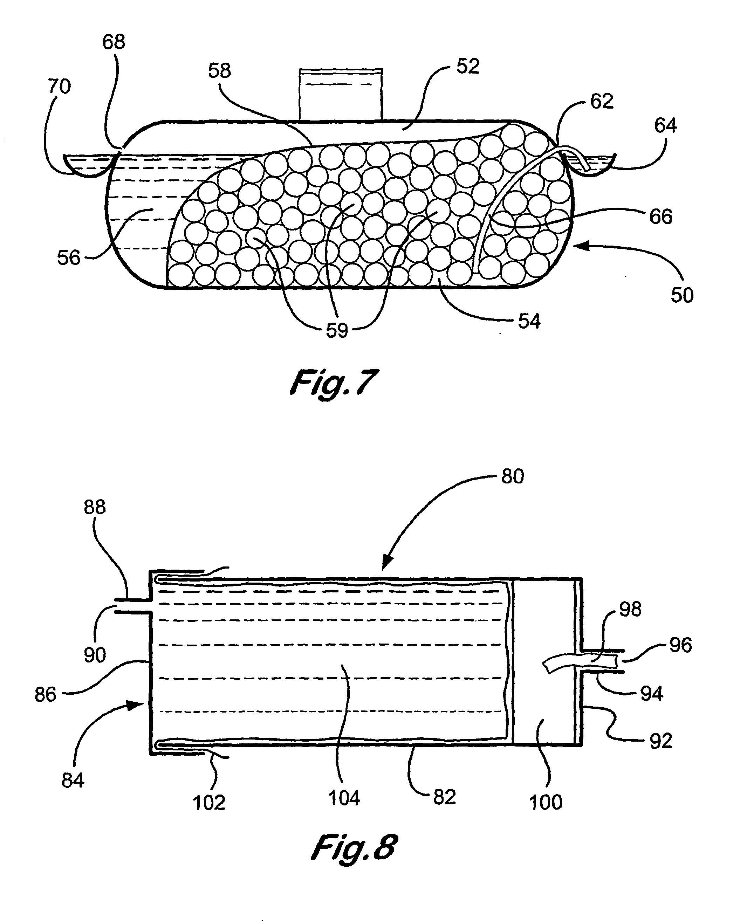

Referring now to Figures 6 and 7, the device thereshown in a lavatory freshener dispenser 50 according to the present invention. The dispenser 50 comprises a chamber 52, which is divided into two sections, 54, 56 by a flexible, waterproof membrane 58. In one section 54 super-absorbent material is contained, and in the other section lavatory freshener is provided 56. The first section, which contains the super-absorbent material 59, further includes an inlet 60, having attached thereto a bowl 64, which can hold a predetermined volume of water. Connecting the bowl and the super-absorbent material is a wick 66.

Connected to the second section 56, holding the lavatory freshener, is an outlet 68. As shown, a second bowl 70 is attached thereto, although this is optional and may not be included in all embodiments.

In use the device is hung over the rim of the lavatory bowl such that it is caught in the flow of water on flushing. When the lavatory is flushed, water from the flush enters the first bowl 64 where it is absorbed by the wick 66 and transferred to the super-absorbent material 59. The volume of water is controlled by the size of the bowl, and also by the length of the flush. The super-absorbent material 59, absorbs the water and expands, pressing against the flexible member 58, reducing the size of the second section 56 of the chamber and thus dispensing a portion of the lavatory freshener into the second bowl. This is then washed into the next flush. As a predetermined volume of water is absorbed by the super-absorbent material, a predetermined volume of lavatory freshener is dispensed into the second bowl 70, as the expansion of the super-absorbent material is dependent upon the volume of water absorbed.

The second bowl is not necessary as the lavatory freshener dispensed by the device can be delivered directly into the lavatory bowl from the dispenser. However, this will be at the end of the flush and after the flush has finished, and thus the lavatory freshener will discharge onto the wall of the lavatory bowl and may discolour it. Thus the second bowl is provided to ensure that the lavatory freshener is added with each flush and does not discharge onto the lavatory bowl wall between flushes.

Referring now to Figure 8, the actuator 80 thereshown is part of a drug delivery system. Earlier drug delivery pumps consist of a syringe driven by a motor. These are subject to mechanical breakdown and interference by electro-magnetic fields, such as generated by mobile phones and other instruments generally used in hospitals for examples MRI scanners.

The drug delivery actuator of the present invention does not require a motor and therefore is not susceptible to the above mentioned problems. The device comprises an elongate rigid container 82. One end 84 of the container is provided with a cap 86, and the cap is provided with an extension 88 forming a liquid outlet 90. The other end 92 of the container 82 has an opening 94 providing a water inlet 96.

To prepare the device for use, a wick 98, typically a piece of cotton string, is placed in the water inlet in the container. Super-absorbent material 100 is placed into the container. A piece of elastomeric material 102 is placed over the end 84 of the container 82, and extending into the container. The elastomeric material 102 is then filled with the fluid 104 to be dispensed. The cap 86 of the container 82 is then fitted over the elastomeric material 102, clamping this in place. Alternatively the cap 86 can be fitted before the fluid 104 to be dispensed is loaded into the elastomeric material 102, which can be achieved through the fluid outlet 90.

As shown super-absorbent material 100 in the form of a powder is used. It is mixed with PVC powder, which helps to wick the water throughout the super-absorbent powder. Alternatively a pad of super-absorbent fibres could be used. In this case a wick is not necessary although may be included. In addition a non-return valve may be included at the water inlet.

When preparing the device for use, it is advantageous to exclude as much air as possible from the container. This helps reduce any likelihood of an air lock or distortion of the dispenser by air.

In use, the fluid outlet 90 is connected by a tube to a hypodermic needle for insertion into a patient (not shown). The water inlet 96 is connected to a suitable water source (not shown). Typically a standard drip arrangement as used in hospitals may be used, but other sources of water supply can be used. The first drops of water are absorbed by the wick 98, and once the wick is fully saturated the water is absorbed by the super-absorbent material 100. As the super-absorbent material absorbs water it expands. This presses of the elastomeric material 102, which in turn expels the fluid 104 through the fluid outlet 90 and into the patient. As the super-absorbent material 100 absorbs the water, to a first approximation, for every one-ml of water absorbed by the super-absorbent material, the super-absorbent material expands by one ml and dispenses one ml of fluid. Thus by adding a set volume of water to the super-absorbent material, a set volume of fluid can be dispensed into a patient.

The rate at which the fluid is dispensed into the patient can also be regulated. The faster the water is added to the super-absorbent material the faster it absorbs the water and expands, displacing the fluid. In addition, pure water is absorbed faster than water containing impurities. Therefore if the fluid is required to be displaced more quickly into a patient, very pure water can be used.

The rate of flow of a drug into a patient is critical to achieve the optimum results for the patient. The above-described pump was tested to ensure that sufficient pressure was generated to pass the drug into the patient, and to ensure that a standard dose could be maintained over a period of time. Pressures generated by the super-absorbent material were in excess of 5 psi, which is high enough for infusion into patients. (Typical blood pressure is approximately 1.5 psi.) It was also demonstrated that the pressure generated by the pump could be maintained over a sufficient period.

## Claims

1. An actuating device (1) for displacing material by movement of a piston (36) or diaphragm (4) the device comprising:
• an enclosure containing unsaturated super-absorbent (39) material (38) and including a piston or diaphragm; and
• a fluid inlet into the enclosure adapted to allow fluid into the enclosure and restrain the super-absorbent material from escaping from the enclosure,
the arrangement being such that as fluid is allowed into the enclosure, it is absorbed by the super-absorbent material which swells against the piston or diaphragm, causing movement thereof and displacement of the material.

2. An actuating device as claimed in claim I, wherein the piston or diaphragm is a membrane of elastomeric material (102) dividing a first section of the enclosure from a second section, one section for holding the unsaturated super-absorbent material, and the other for holding liquid or the like to be displaced.

3. An actuating device as claimed in claim 1, wherein the piston or diaphragm is a bag forming a second section within the enclosure, and sealed at or near an outlet of the enclosure, the bag being fillable with liquid to be displaced.

4. An actuating device as claimed in claim 1, claim 2 or claim 3, further including means for controlling the amount of fluid added to the inlet to control the expansion of the super-absorbent material.

5. An actuating device as claimed in claim 2, or claim 4 as appendant to claim 2, wherein the second section includes an opening (90) through which the material is displaced.

6. An actuating device as claimed in any preceding claim, wherein the super-absorbent material is in granular form.

7. An actuating device as claimed in any one of claims 1 to 5, wherein the super-absorbent material is in fibrous form.

## Patentansprüche

1. Betätigungsmittel (1) zum Verdrängen von Material durch Bewegung eines Kolbens (36) oder einer Membran (4),
wobei das Mittel aufweist:
■ eine Umhüllung, die ein ungesättigtes, hochabsorbierendes Material (38) enthält und einen Kolben oder eine Membran aufweist; und
■ einen Fluideinlaß in die Umhüllung, der so ausgebildet ist, daß er einen Flüssigkeitseintritt in die Umhüllung erlaubt und einen Austritt des hoch-absorbierenden Materials aus der Umhüllung unterbindet,
wobei die Anordnung so getroffen ist, daß Flüssigkeit nach ihrem Einlaß in die Umhüllung von dem hoch-absorbierenden Material aufgenommen wird, das gegen den Kolben oder die Membran anschwillt und dessen bzw. deren Bewegung und das Verdrängen des Materials bewirkt.

2. Betätigungsmittel nach Anspruch 1, worin der Kolben oder die Membran eine Membran aus gummiartigem Werkstoff (102) ist, die einen ersten Bereich der Umhüllung von einem zweiten Bereich abteilt, wobei der eine Bereich das ungesättigte hoch-absorbierende Material aufnimmt und der andere Bereich die zu verdrängende Flüssigkeit oder dergleichen enthält.

3. Betätigungsmittel nach Anspruch 1, worin der Kolben oder die Membran ein einen zweiten Bereich innerhalb der Umhüllung bildender Beutel ist, der an oder nahe einem Auslaß der Umhüllung abgedichtet ist, wobei der Beutel mit zu verdrängender Flüssigkeit befüllbar ist.

4. Betätigungsmittel nach Anspruch 1, Anspruch 2 oder Anspruch 3, ferner aufweisend Mittel zum Steuern bzw. Regeln der dem Einlaß zugeführten Flüssigkeitsmenge zum Steuern bzw. Regeln der Expansion des hoch-absorbierenden Materials.

5. Betätigungsmittel nach Anspruch 2 oder Anspruch 4 in Rückbezug auf Anspruch 2, worin der zweite Bereich eine Öffnung (90) aufweist, durch die das Material verdrängt wird.

6. Betätigungsmittel nach einem vorstehenden Anspruch, worin das hochabsorbierende Material körnerförmig ist.

7. Betätigungsmittel nach einem der Ansprüche 1 bis 5, worin das hochabsorbierende Material faserförmig ist

## Revendications

1. Dispositif d'actionnement (1) destiné à déplacer un matériau grâce au déplacement d'un piston (36) ou d'un diaphragme (4), le dispositif comprenant :
■ une enceinte contenant un matériau superabsorbant insaturé (38) et comprenant un piston ou diaphragme; et
■ une entrée de fluide dans l'enceinte adaptée pour laisser pénétrer du fluide dans l'enceinte et empêcher le matériau superabsorbant de s'échapper de l'enceinte,
le dispositif étant agencé de telle sorte qu'au fur et à mesure que le fluide est admis dans l'enceinte, il est absorbé par le matériau superabsorbant qui gonfle contre le piston ou diaphragme, entraînant le déplacement de celui-ci et le déplacement du matériau.

2. Dispositif d'actionnement selon la revendication 1, dans lequel le piston ou diaphragme est une membrane en matériau élastomère (102) séparant une première section de l'enceinte d'une deuxième section, une section servant à contenir le matériau super absorbant insaturé, et l'autre pour contenir le liquide ou similaire à déplacer.

3. Dispositif d'actionnement selon la revendication 1, dans lequel le piston ou diaphragme est un sac formant une deuxième section à l'intérieur de l'enceinte, et fermé au niveau d'une sortie de l'enceinte, ou à proximité de celle-ci, le sac pouvant être rempli par la liquide à déplacer.

4. Dispositif d'actionnement selon la revendication 1, la revendication 2 ou la revendication 3, comprenant, en outre des moyens pour commander la quantité de fluide ajouté par l'entrée afin de commander l'expansion du matériau superabsorbant.

5. Dispositif d'actionnement selon la revendication 2, ou la revendication 4 lorsqu'elle est dépendante de la revendication 2, dans lequel la deuxième section comprend une ouverture (90) par laquelle la matériau est déplacé.

6. Dispositif d'actionnement selon l'une quelconque des revendications précédentes, dans lequel le matériau superabsorbant se présente sous forme de granulés.

7. Dispositif d'actionnement selon l'une quelconque des revendications 1 à 5, dans lequel le matériau superabsorbant se présente sous forme de fibres.
